# EUROPÄISCHE PATENTSCHRIFT

(11) **EP 1 313 688 B1**
(45) Veröffentlichungstag und Bekanntmachung des Hinweises auf die Patenterteilung: **03.11.2004**
(21) Anmeldenummer: 01967255.9
(22) Anmeldetag: 10.08.2001
(51) Int. Cl.: C07C 29/38, C07C 31/22, C07C 51/16, C07C 53/06, C07C 29/76

(54) **TMP/DAMPFDRUCKFILTRATION**
TMP/STEAM PRESSURE FILTRATION
PRODUCTION DE TRIMETHYLOLPROPANE PAR FILTRATION SOUS PRESSION DE VAPEUR

(30) Priorität: 23.08.2000 DE 10041197
(43) Veröffentlichungstag der Anmeldung: 28.05.2003
(73) Patentinhaber: Bayer Chemicals AG, 51368 Leverkusen (DE)
(72) Erfinder: WAGNER, Paul, 40597 Düsseldorf (DE); KLAUSENER, Alexander, 50259 Pulheim (DE)
(86) Internationale Anmeldenummer: PCT/EP2001/009255
(87) Internationale Veröffentlichungsnummer: WO 2002/016294

(56) Entgegenhaltungen:
- US-A- 2 468 718
- CHEMICAL ABSTRACTS, vol. 70, no. 25, 23. Juni 1969 (1969-06-23) Columbus, Ohio, US; abstract no. 114517z, M.P. VYSOTSKII, ET AL.: "Removal of sodium formate and formic acid impurities from aqueous and nonaqueous solutions of the condensation products of aliphatic aldehydes and formaldehyde on ion-exchange resins" Seite 275; XP002184533 & KARBONILIROVANIE NENASYSHCHENNYKH UGLEVODORODOV, 1968, Seiten 263-270,
- R. BOTT, ET AL.: "Dampf-Druckfiltration - die fortschrittliche Version der kontinuierlichen Druckfiltration (Hi-Bar Filtration)" AUFBEREITUNGS-TECHNIK, Bd. 37, Nr. 4, April 1996 (1996-04), Seiten 163-170, XP000583092 Verlag für Aufbereiting Schirmer und Zeh, Wiesbaden, DE ISSN: 1443-9302 in der Anmeldung erwähnt

## Beschreibung

Die vorliegende Erfindung betrifft ein Verfahren zur Herstellung von Trimethylolpropan unter gleichzeitiger Bildung eines Formiatsalzes und die Abtrennung des Formiatsalzes aus dem Reaktionsgemisch, ggf. nach vorheriger Einengung dieses Reaktionsgemisches.

Sowohl Trimethylolalkane, als auch Formiatsalze sind industriell interessante Produkte. So findet Trimethylolpropan Verwendung im Bereich der Herstellung von Lackharzen, Pulverlacken, Schaumstoffen und verschiedensten Polyestern. Formiatsalze lassen sich beispielsweise zur Gewinnung von Ameisensäure nutzen. Calciumformiat ist besonders vielseitig verwendbar und wird beispielsweise in folgenden Bereichen kommerziell eingesetzt: Zusatzstoff im Bereich der Tierernährung (Schweine-, Rinder- und Putenmast), Einsatz im Bereich der Baustoffindustrie (Verbesserung der Aushärtung von Zement, Gips und Fugenmassen sowie Frostschutzmittel für Mörtel), Hilfsstoff in der Lederindustrie, Hilfsmittel bei der Herstellung von Hochglanzpapieren, Behandlung von Waschwässern bei der Rauchgasentschwefelung, Hilfsmittel bei der Silierung.

Die industrielle Herstellung von Trimethylolpropan (TMP) wird gegenwärtig nach dem sogenannten anorganischen Cannizzaro-Verfahren durchgeführt, ein Verfahren, das unter gleichzeitiger Gewinnung eines Formiatsalzes abläuft. Dieses Verfahren geht von n-Butyraldehyd und Formaldehyd als Ausgangsmaterialien aus. Nach allgemeinem Verständnis wird zunächst in einer basenkatalysierten Aldolreaktion über die Zwischenstufe 2-Methylolbutanal 2,2-Dimethylolbutanal gebildet. Unter Einwirkung *stöchiometrischer* Mengen einer Base, bevorzugt wird Natrium- oder Calciumhydroxid eingesetzt, erfolgt im abschließenden Schritt, einer gekreuzten Cannizzaro-Reaktion, die Bildung von Trimethylolpropan unter gleichzeitiger Freisetzung der entsprechenden Formiatsalze. Beispielsweise entsteht Calciumformiat, wenn Calciumhydroxid als Base eingesetzt wird.

Die entstehenden Formiatsalze müssen vor der destillativen Aufarbeitung zur Gewinnung des Trimethylolpropans möglichst vollständig aus dem Reaktionsgemisch abgetrennt werden, da Formiatsalze unter Destillationsbedingungen die Zersetzung des Trimethylolpropans katalysieren. Andererseits muss das Formiatsalz von organischen Resten befreit und getrocknet werden, um einer Verwendung zugeführt werden zu können. Zur Abtrennung des Formiatsalzes sind zahlreiche Verfahren bekannt.

So kann gemäß Ullmann's Enzyclopedia of Industrial Chemistry, 5. Auflage 1985, S. 315 aus der aufkonzentrierten Reaktionslösung Trimethylolpropan mit einem geeigneten organischen Lösungsmittel extrahiert werden. Das Lösungsmittel wird anschließend im Vakuum entfernt und das erhaltene Roh-Trimethylolpropan destillativ gereinigt. Alternativ kann die wässrige Reaktionslösung so weit eingeengt werden, dass das Formiat kristallisiert. Anschließend wird zur Entfernung des Kristallisats heiß filtriert.

In der deutschen Auslegeschrift DE-AS 1 052 383 wird ein Verfahren beschrieben, in dem die Abtrennung des Trimethylolpropans durch Abtreiben des Trimethylolpropans mittels überhitztem Wasserdampf in einem Dünnschichtverdampfer erfolgt. Das Verfahren wird zweckmäßigerweise bei erniedrigtem Druck und Wasserdampftemperaturen von 120 °C bis 220 °C durchgeführt.

DE-A 32 07 746 beschreibt ein Verfahren zur Herstellung von Trimethylolpropan unter Verwendung von Formaldehyd mit einem besonders niedrigen Methanolgehalt. Zur Aufarbeitung des Reaktionsgemischs wird diesem Säure zugegeben, so dass sich ein pH-Wert von 5-7 einstellt. Anschließend wird überschüssiger Formaldehyd destillativ abgetrennt. Die Isolierung des Trimethylolpropans erfolgt durch Destillation oder Extraktion.

Die genannten Verfahren zeigen technologische Schwierigkeiten im Zusammenhang mit der Auftrennung von Reaktionsgemischen, die bei der Herstellung von Trimethylolpropan nach dem Cannizzaro-Verfahren anfallen. Zur Abtrennung der entstehenden Formiate ist noch keine optimale Lösung gefunden worden. Dies lässt sich auch daran erkennen, dass gerade in jüngster Zeit große Anstrengungen unternommen wurden, Verfahren zur Herstellung von Trimethylolpropan zu entwickeln oder zu verbessern, die ohne Anfall von Alkali- oder Erdalkaliformiaten im Produktgemisch verlaufen.

Laut DE-A 1 952 738 lässt sich die Umsetzung von n-Butyraldehyd und Formaldehyd in Gegenwart eines niederen tertiären Amins durchführen. Es wird ein etwa 6-facher Überschuss an Formaldehyd und ein etwa 1,5-facher Überschuss Trialkylamin eingesetzt. Neben dem gewünschten Produkt Trimethylolpropan werden stöchiometrische Mengen an Trialkylammoniumformiat erhalten, das leicht destillativ aus dem Reaktionsgemisch entfernt werden kann. Anders als Alkalimetall- oder Erdalkalimetallformiate, ist dieses jedoch nicht weiter verwertbar, so dass vorgeschlagen wird, das Trialkylammoniumformiat in einem separaten Reaktionsschritt in Calciumformiat umzuwandeln und das dabei freiwerdende Amin in den Kreislauf zurückzuführen. Dieses Vorgehen macht das Verfahren wirschaftlich wenig sinnvoll.

Gemäß DE-A 196 53 093 wird zunächst 2,2-Dimethylolbutanal durch Kondensation von n-Butyraldehyd und Formaldehyd in Gegenwart katalytischer Mengen eines tertiären Amins in drei Stufen hergestellt, wobei nicht umgesetztes Edukt und gebildete Nebenprodukte rezykliert und weiter umgesetzt werden. Das auf diese Weise erhaltene Kondensationsprodukt (2,2-Dimethylolbutanal) wird anschließend zu Trimethylpropan hydriert. Auf die Gewinnung eines verwertbaren Formiats wird bei diesem Verfahren verzichtet.

Auch EP-A 860 419 schlägt vor, die Herstellung von 2,2-Dimethylolbutanal aus n-Butyraldehyd und Formaldehyd mehrstufig durchzuführen, wobei in der ersten Stufe die eigentliche Umsetzung erfolgt und in der zweiten Stufe das als Nebenprodukt anfallende 2-Ethylacrolein mit weiterem Formaldehyd umgesetzt wird. Das so hergestellte 2,2-Dimethylolbutanal kann im Folgenden zu Trimethylolpropan hydriert werden, wobei wiederum keine Gewinnung von Formiat erfolgt.

Die bekannten Verfahren zur Herstellung von Trimethylolpropan ohne Bildung eines Formiatsalzes sind bislang dem anorganischen Cannizzaro-Verfahren unterlegen. Das liegt zum einen daran, dass Formiatsalze interessante und vermarktbare Produkte darstellen, zum anderen daran, dass eine Hydrierreaktion nur in speziell dafür ausgelegten teuren Apparaturen durchgeführt werden kann.

Die Aufgabe der vorliegenden Erfindung bestand darin, ein Verfahren zur gleichzeitigen Herstellung von Trimethylolpropan und eines Formiatsalzes und zur effektiven Isolierung beider Produkte bereitzustellen, das eine hohe Raum-Zeit-Ausbeute erlaubt und beide Produkte in möglichst reiner Form liefert. Diese Aufgabe wird mittels des erfindungsgemäßen Verfahrens gelöst.

Die Erfindung betrifft ein Verfahren zur Herstellung von Trimethylolpropan unter gleichzeitiger Bildung von Formiatsalzen der Formel

M(OOCH)ₙ (I),

wobei M für die Alkalimetalle Lithium, Natrium, Kalium, Rubidum, Caesium und/oder die Erdalkalimetalle Beryllium, Calcium, Strontium, Barium steht und n für den Fall, dass M ein Alkalimetall ist, 1, und für den Fall, dass M ein Erdalkalimetall ist, 2 bedeutet, dadurch gekennzeichnet, dass n-Butyraldehyd, Formaldehyd und Base nach dem anorganischen Cannizzaro-Verfahren umgesetzt werden und man das entstehende Reaktionsgemisch, der Dampfdruckfiltration unterwirft.

Gegebenenfalls werden vor der Dampfdruckfiltration destillierbare Bestandteile aus dem Reaktionsgemisch teilweise abgetrennt.

Gegebenenfalls können nach der Dampfdruckfiltration weitere Reinigungs- und Trocknungsschritte durchgeführt werden.

Das erfindungsgemäße Verfahren zeichnet sich durch gute Raum-Zeit-Ausbeuten aus. Für das isolierte Formiat werden Reinheits- und Trocknungsgrade erreicht, die mittels einfacher Filtration, Zentrifugation oder Verdampfungskristallisation nicht erreicht werden. Die isolierten Formiatsalze sind weitestgehend von organischen Anhaftungen und von Feuchtigkeit befreit, ohne dass dabei mehrere Verfahrensschritte in räumlich getrennten Apparaturen hintereinandergeschaltet werden müssen.

Das prinzipielle Verfahren der Dampfdruckfiltration ist in DE-C 42 38 087 und in R. Bott, Th. Langeloh, Aufarbeitungstechnik 37, Nr.4, 1996, 163-170 beschrieben. Als Anwendungsgebiete sind die Bereiche Filtration von Kohleschlämmen, Pigmentfiltration, Stärkefiltration, Zuckerfiltration und Hydrometallurgie genannt. Es werden also immer unlösliche Feststoffe aus einer Suspension abgetrennt. Überraschenderweise lässt sich die Dampfdruckfiltration auch für eine äußerst effektive und qualitativ befriedigende Abtrennung von Formiatsalzen aus den Gemischen, die im Zuge der Herstellung und Isolierung von Trimethylolpropan nach dem anorganischen Cannizzaro-Verfahren entstehen, nutzen. Das war nicht ohne Weiteres zu erwarten, da bei der Dampfdruckfiltration der gebildete Filterkuchen mit einem dampfförmigen Behandlungsfluid, vorzugsweise Wasserdampf, beaufschlagt wird und somit zu befürchten war, dass nennenswerte Mengen des Formiatsalzes gelöst würden. Reste an Formiatsalzen in Trimethylolpropan-haltigen Filtraten haben jedoch die bereits beschriebenen Nachteile. Außerdem mußte befürchtet werden, daß angesichts der vergleichsweise hohen Dampftemperaturen nachteilige Einflüsse auf die Qualität des isolierten Formiatsalzes, bedingt etwa durch thermische Zersetzung von im zu filtrierenden Gemisch enthaltenen organischen Komponenten, wirksam werden würden.

Entsprechend des erfindungsgemäßen Verfahrens wird zunächst n-Butyraldehyd mit Formaldehyd in Gegenwart einer Base umgesetzt. Diese Umsetzung kann in einer oder mehreren Stufen durchgeführt werden. Bei der einstufigen Variante werden die Edukte direkt zu Trimethylolpropan und einem Formiatsalz umgesetzt. Bei einer zweistufigen Variante wird zunächst n-Butyraldehyd und Formaldehyd in Gegenwart katalytischer Mengen Base zu 2,2-Dimethylolbutanal umgesetzt, gegebenenfalls Reinigungs- oder Separationsschritte durchgeführt und dann erst das 2,2-Dimethylolbutanal mit weiterem Formaldehyd in Gegenwart stöchiometrischer Mengen an Base zu Trimethylolpropan und Formiatsalz umgesetzt.

Nach dem erfindungsgemäßen Verfahren werden neben Trimethylolpropan die Formiatsalze der Formel

M(OOCH)ₙ (I)

erhalten, wobei M für die Alkalimetalle Lithium, Natrium, Kalium, Rubidum, Caesium und/oder die Erdalkalimetalle Beryllium, Calcium, Strontium, Barium steht und n für den Fall, dass M ein Alkalimetall ist, 1, und für den Fall, dass M ein Erdalkalimetall ist, 2 bedeutet.

Bevorzugt steht M für Natrium, Kalium, Rubidium, Caesium, Calcium, Strontium und/oder Barium, besonders bevorzugt für Natrium, Kalium, Caesium und/oder Calcium und insbesondere bevorzugt für Natrium und/oder Calcium.

Geeignete Basen sind Verbindungen aus der Reihe der Alkali- und Erdalkalimetallhydroxide, Alkali- und Erdalkalimetallhydrogencarbonate und der Alkali- und Erdalkalimetallcarbonate, wobei die eingesetzte Base naturgemäß das Alkali- oder Erdalkaliion enthalten muss, das im gewünschten Formiatsalz enthalten sein soll. Bevorzugte Basen sind Natriumhydroxid, Calciumhydroxid, Natriumhydrogencarbonat und Natriumcarbonat. Die Verbindungen können jeweils alleine, aber auch als Gemische aus zwei oder mehreren basischen Komponenten der aufgeführten Substanzen eingesetzt werden. Es empfiehlt sich jedoch, nur Basen mit identischem Kation einzusetzen, um ein einheitliches Formiatsalz zu erhalten.

Formaldehyd wird vorzugsweise in Form einer wässrigen Lösung eingesetzt, die beispielsweise etwa 5 bis 99 Gew.-%, bevorzugt 5 bis 75 Gew.-%, besonders bevorzugt 10 bis 55 Gew.-% Formaldehyd enthält. Insbesonders bevorzugt wird Formaldehyd in der Form eingesetzt, in der er bei üblichen großtechnischen Prozessen zur Herstellung von Formaldehyd anfällt.

Das Molverhältnis zwischen n-Butyraldehyd und Formaldehyd beträgt beispielsweise 1:2 bis 1:10, bevorzugt 1:2 bis 1:5, besonders bevorzugt 1:2 bis 1:4.

Die Umsetzung kann im Allgemeinen bei einer Temperatur von 0 bis 100°C, bevorzugt bei 10 bis 90°C, besonders bevorzugt bei 15 bis 70°C durchgeführt werden.

Die Verweilzeit des Reaktionsgemisches im Reaktor kann in der Regel 0,15 bis 10 Stunden betragen.

Die Umsetzung kann sowohl batchweise, semibatch als auch kontinuierlich betrieben werden. Als Reaktionsapparate kommen alle dem Fachmann bekannten Reaktionsapparate, die zur Umsetzung von flüssigen Reaktanden geeignet sind, in Frage. Besonders erwähnt sei der Rührkesselreaktor, die Rührkesselkaskade, das Strömungsrohr und der Mehrkammerreaktor.

Das so erhaltene Produktgemisch aus Trimethylolpropan, Formiatsalz und gegebenenfalls weiteren Komponenten, wie Wasser, Alkohole, unumgesetzte Edukte und gebildete Nebenprodukte, wird, gegebenenfalls nach teilweiser Abtrennung destillierbarer Bestandteile, als Flüssig-Fest-Suspension erfindungsgemäß einer Dampfdruckfiltration unterworfen, wobei das Formiatsalz aus dem Gemisch abgetrennt, von organischen Verunreinigungen befreit und entfeuchtet wird. Gegebenenfalls können dieser Prozedur weitere nachgeschaltete Reinigungs- und Trocknungsschritte folgen.

Bevorzugt handelt es sich bei der genannten teilweisen Abtrennung destillierbarer Bestandteile um das teilweise Abdestillieren von Wasser sowie das teilweise oder vollständige Abdestillieren weiterer flüchtiger Komponenten wie beispielsweise unumgesetzter Ausgangsstoffe, leichtersiedender Nebenprodukte oder geringer Anteile des gebildeten Produktes Trimethylolpropan.

Geeignete Filtrationsapparate sind beispielsweise in DE-C 42 38 087 beschrieben. Im wesentlichen besteht ein solcher Filtrationsapparat aus einer in einem Gehäuse angeordneten Filterfläche, wobei die Filterfläche von einem als Druckraum ausgebildeten druckdichten Gehäuse umgeben ist. Der Druckraum ist mit einem unter Überdruck stehenden Gas gefüllt. Zumindest ein Abschnitt der Filterfläche ist von einer Haube umgeben, die zusammen mit der Filterfläche einen zweiten Druckraum umschließt, der das Bedampfungsfluid in gasförmigem Zustand enthält. Der Filtrationsapparat enthält zur Waschung des Filterkuchens vorgesehene Waschvorrichtungen, vorzugsweise Rohre und Düsen, die sowohl unter der Haube, als auch davor oder danach über der Filterfläche angeordnet sein können.

Die Abtrennung von Formiatsalzen gemäß dem erfindungsgemäßen Verfahren kann sowohl kontinuierlich, als auch diskontinuierlich erfolgen. Für den kontinuierlichen Betrieb können beispielsweise Trommel-, Scheiben-, Band- oder Tellerfilter eingesetzt werden. Für den diskontinuierlichen Betrieb eignen sich beispielsweise Nutschenfilter. Bevorzugt wird die Abtrennung kontinuierlich unter Verwendung eines rotierenden Trommel- oder Scheibenfilters, besonders bevorzugt unter Verwendung eines rotierenden Trommelfilters durchgeführt.

Das der Dampfdruckfiltration zugeleitete zu filtrierende Gemisch enthält im Allgemeinen beispielsweise 5 bis 95 Gew.-%, bevorzugt 6 bis 90 Gew.-%, besonders bevorzugt 7 bis 85 Gew.-% und insbesondere bevorzugt 8 bis 75 Gew.-% Trimethylolpropan. Es enthält des Weiteren beispielsweise 5 bis 95 Gew.-%, bevorzugt 6 bis 90 Gew.-%, besonders bevorzugt 7 bis 85 Gew.-% und insbesondere bevorzugt 8 bis 75 Gew.-% des Formiatsalzes und 5 bis 95 Gew.-%, bevorzugt 6 bis 90 Gew.-%, besonders bevorzugt 7 bis 85 Gew.-% und insbesondere bevorzugt 8 bis 80 Gew.-% Wasser. Darüberhinaus können weitere Komponenten, beispielsweise nicht umgesetzte Edukte oder organische Verunreinigungen, enthalten sein.

Die Temperatur des der Dampfdruckfiltration zugeleiteten Gemischs kann beispielsweise zwischen 0 °C und 150 °C, bevorzugt zwischen 5 °C und 120 °C und besonders bevorzugt zwischen 10 °C und 110 °C liegen.

Bei der Dampfdruckfiltration werden in der Regel 4 Phasen durchlaufen, nämlich Kuchenbildung, Kuchenwaschung, Kuchenbedampfung und Kuchenentfeuchtung. Dabei kann die Kuchenwaschung vor, während und/oder nach der Kuchenbedampfung erfolgen. Es ist jedoch auch möglich, die Dampfdruckfiltration ohne Kuchenwaschung durchzuführen, da die Kuchenbedampfung ebenfalls eine Waschung bewirkt. Vorzugsweise wird die Phase der Kuchenwaschung durchlaufen.

Das bei der Herstellung von Trimethylolpropan nach dem anorganischen Cannizzaro-Verfahren anfallende Reaktionsgemisch wird, gegebenenfalls nach einer Voreindampfung zum Abtrennen leichtsiedender Verunreinigungen, wie beispielsweise nicht umgesetzter Formaldehyd, und eines Teils des im Reaktionsgemisch enthaltenen Wassers, der Dampfdruckfiltrations-Apparatur zugeführt. Auf der Filterfläche bildet sich ein Filterkuchen aus dem Formiatsalz aus.

Während der Kuchenbildungsphase ist es vorteilhaft, einen Differenzdruck anzulegen. Dieser liegt beispielsweise zwischen 0 und 6 bar, bevorzugt zwischen 0,3 und 4 bar, besonders bevorzugt zwischen 0,5 und 3 bar.

Die Kuchenbildungsphase dauert im Allgemeinen zwischen 2 und 420 Sekunden, bevorzugt zwischen 3 und 360 Sekunden und besonders bevorzugt zwischen 4 und 300 Sekunden.

Als Filtermedien können beispielsweise Metallgewebe oder temperaturbeständige Kunststoffmaterialien eingesetzt werden.

An die Kuchenbildung kann sich in beliebiger Reihenfolge Kuchenwaschung und Kuchenbedampfung anschließen, vorzugsweise wird zunächst die Kuchenwaschung durchgeführt.

Die Kuchenwaschung kann aus einer oder mehreren Waschvorgängen bestehen. Dazu wird der Filterkuchen mit einer oder mehreren gegebenenfalls verschiedenen Waschflüssigkeiten beaufschlagt. Zum Waschen eines erfindungsgemäß gewonnenen Formiatsalz-Kuchens haben sich insbesondere folgende Waschflüssigkeiten bewährt: Reines Wasser und/oder Dampf bzw. dessen Kondensat, und/oder das zu gewinnende Formiatsalz enthaltendes Wasser, bevorzugt mit dem zu gewinnenden Formiatsalz teilgesättigtes, gesättigtes oder übersättigtes Wasser und/oder ein bei der Dampfdruckfiltration anfallendes Filtrat, wobei die Waschflüssigkeiten in reiner Form oder in beliebiger Mischung eingesetzt werden können.

Vorteilhaft werden die Filtrate aus verschiedenen Bereichen des Filters getrennt abgeführt und separat aufgefangen. Die Filtrate können dann gezielt einer Weiterverarbeitung zugeführt werden. Zur Optimierung der Waschung ist es, wie bereits erwähnt vorteilhaft, eines oder mehrere dieser Filtrate als Waschflüssigkeit in die Dampfdruckfiltration zurückzuführen.

Die Temperaturen der Waschflüssigkeiten liegen beispielsweise zwischen 0 °C und 165 °C, bevorzugt zwischen 5 °C und 155 °C und besonders bevorzugt zwischen 10 °C und 145 °C.

Der Differenzdruck am Filterkuchen während der Kuchenwaschung beträgt beispielsweise zwischen 0 und 6 bar, bevorzugt zwischen 0,3 und 4 bar, besonders bevorzugt zwischen 0,5 und 3 bar.

Die Kuchenwaschungsphase dauert beispielsweise zwischen 0 und 420 Sekunden, bevorzugt zwischen 1 und 360 Sekunden und besonders bevorzugt zwischen 2 und 300 Sekunden.

Bei der Kuchenbedampfung wird der auf der Filterfläche gebildete Filterkuchen aus dem Formiatsalz mit einem dampfförmigen Behandlungsfluid beaufschlagt, dessen Druck und Temperatur derart eingestellt werden, dass das Behandlungsfluid im Filterkuchen kondensiert, vorzugsweise zunächst in der obersten, von der Filterfläche abgewandten Schicht des Filterkuchens, so dass entlang dieser Schicht eine Kondensationsfront gebildet wird und dass weiterhin der Filterkuchen von der obersten Schicht aus in Richtung der Filterflächen auf die Temperatur des gasförmigen Behandlungsfluids fortschreitend erwärmt wird, so dass dabei die Kondensationsfront an der Phasengrenze zwischen dem gasförmigen Behandlungsfluid und der Flüssigkeit zur Filterfläche hin verschoben wird. Die gasförmige Phase des Behandlungsfluids kann dabei als Druckmedium zur Erzeugung eines Differenzdruckes verwendet werden. Der Kuchen kann durch die Kuchenbedampfung nochmals gewaschen, aufgeheizt und mechanisch und thermisch entfeuchtet werden.

Als Behandlungsfluid wird beispielsweise deionisiertes Wasser verwendet. Es können aber auch andere Behandlungsfluide verwendet werden, beispielsweise organische Lösungsmittel.

Die Temperatur des Dampfes, der der Dampfdruckfiltration zugeleitet wird, kann beispielsweise zwischen 100 °C und 250 °C, bevorzugt zwischen 105 °C und 220 °C und besonders bevorzugt zwischen 110 °C und 200 °C liegen.

Der Differenzdruck am Filterkuchen liegt während der Kuchenbedampfung beispielsweise zwischen 0 und 6 bar, bevorzugt zwischen 0,3 und 4 bar, besonders bevorzugt zwischen 0,5 und 3 bar.

Die Kuchenbedampfungsphase dauert beispielsweise zwischen 0,5 und 300 Sekunden, bevorzugt zwischen 0,5 und 240 Sekunden und besonders bevorzugt zwischen 0,5 und 180 Sekunden.

Nach Kuchenwaschung und Kuchenbedampfung kann sich die Kuchenentfeuchtung anschließen. Dabei wird der Filterkuchen beispielsweise mit überhitztem Wasserdampf, mit komprimiertem Inertgas und/oder komprimierter Luft mechanisch und thermisch entfeuchtet. Bevorzugt wird zur Entfeuchtung des Formiatsalz-Kuchens komprimierte und gegebenenfalls temperierte Luft verwendet.

Der Differenzdruck in der Kuchenentfeuchtungsphase liegt beispielsweise zwischen 0 und 6 bar, bevorzugt zwischen 0,3 bis 4 bar, besonders bevorzugt zwischen 0,5 und 3 bar.

Die Temperatur des komprimierten Inertgases bzw. der komprimierten Luft liegt beispielsweise zwischen 0 °C und 250 °C, bevorzugt zwischen 10 °C und 220 °C und besonders bevorzugt zwischen 20 °C und 200 °C.

Die Kuchenentfeuchtungsphase dauert beispielsweise zwischen 0 und 300 Sekunden, bevorzugt zwischen 0,5 und 260 Sekunden und besonders bevorzugt zwischen 0,5 und 180 Sekunden.

Der gewaschene, bedampfte und entfeuchtete Formiat-Filterkuchen kann vom Filtertuch abgenommen und über eine Kuchenaustragsschleuse aus dem Druckraum herausgefördert werden.

Das so gewonnene Formiatsalz weist in der Regel einen Restfeuchtegehalt von 0,1-3,5 Gew.-%, bevorzugt von 0,1-3,0 Gew.-% auf. Der Gehalt an organischen Verunreinigungen beträgt beispielsweise < 500 ppm, bevorzugt < 300 ppm.

Im Gegensatz zu den bislang bekannten Verfahren zur Aufarbeitung von Gemischen, die Trimethylolpropan und ein Formiatsalz enthalten, erlaubt die Dampfdruckfiltration nicht nur die Abtrennung des Formiatsalzes aus im Zuge der Herstellung und Isolierung von Trimethylolpropan anfallenden Gemischen, sondern auch die Entfernung von anhaftenden organischen Rückständen und die Entfeuchtung des Formiatsalzes. Dies führt zu einer weiteren Verbesserung der Reinheit des Formiatsalzes.

Die bei der Dampfdruckfiltration gewonnenen Trimethylolpropan-haltigen Filtrate sind weitgehend von Formiatsalzen frei, so dass das Trimethylolpropan auf an sich bekannte Weise isoliert werden kann. Die Aufarbeitung der Filtrate erfolgt in der Regel destillativ.

Das anfallende Dampfkondensat und die Waschfiltrate können, gegebenenfalls nach vorheriger Aufarbeitung, einer geregelten Entsorgung zugeführt werden. Wirtschaftlich sinnvoller kann es jedoch sein, diese teilweise oder ganz, kontinuierlich oder diskontinuierlich, an geeigneter Stelle, beispielsweise als Waschflüssigkeit, in den Prozess zurückzuführen.

Das erhaltene Formiatsalz kann in der ausgetragenen Form weiterverwendet, gegebenenfalls aber auch noch einem oder mehreren weiteren isolierten oder kombinierten Reinigungs- und Trocknungsschritten zugeführt werden. Dafür kommen alle bekannten und für den vorliegenden Fall geeigneten Reinigungs- und Trocknungsverfahren in Frage. An Reinigungsverfahren seien beispielhaft genannt: Umkristallisation aus Wasser oder einem Wasser enthaltenden Lösungsmittelgemisch oder eine Redispergierung in Wasser oder einem Wasser enthaltenden Lösungsmittelgemisch. An Trocknungsverfahren seien beispielhaft genannt: Konvektionstrocknung (Umlufttrocknung, Bandtrocknung, Wirbelstromtrocknung, Stromtrocknung oder Zerstäubungstrocknung), Kontakttrocknung (Tellertrockner, Doppelkonustrockner, Schaufeltrockner), Gefriertrocknung oder Strahlungtrocknung.

Das folgende Beispiel soll das erfindungsgemäße Verfahren weiter verdeutlichen, ohne dass darin eine Einschränkung des erfindungsgemäßen Gedankens zu sehen wäre.

### Beispiel

240 g eines Gemisches, enthaltend ca. 45 Gew.-% Trimethylolpropan, ca. 28 Gew.-% Calciumformiat, ca. 20 % Wasser sowie weitere organische Komponenten wurde einer Labordruckfilterzelle mit einer Filterfläche von 22 cm² zugeführt (Filtermaterial: Polypropylenfiltertuch, 25 µm). Mittels Druckluft wurde auf die zu filtrierende Masse ein Druck von 2 bar aufgebracht, der Druck auf der Filtratseite betrug ca. 1 bar. Die Kuchenbildungszeit nahm 26 Sekunden in Anspruch. Nachfolgend wurde während 25 Sekunden mit 80 g einer gesättigten wäßrigen Lösung von Calciumformiat auf dem Filter gewaschen. Danach wurde während 9,3 Sekunden eine Beaufschlagung des Filterkuchens mit auf 2 bar entspanntem Dampf durchgeführt. Anschließend erfolgte während 26 Sekunden eine Trocknung des heißen Filterkuchens mit Druckluft.

Es wurden 65 g Calciumformiat erhalten, die eine Restfeuchte von 1,95 Gew.-% (K. Fischer) und einen Restorganikagehalt von 77 mg/kg aufwiesen.

## Patentansprüche

1. Verfahren zur Herstellung von Trimethylolpropan unter gleichzeitiger Bildung von Formiatsalzen der Formel
M(OOCH)ₙ (I),
wobei M für die Alkalimetalle Lithium, Natrium, Kalium, Rubidum, Caesium und/oder die Erdalkalimetalle Beryllium, Calcium, Strontium, Barium steht und n für den Fall, dass M ein Alkalimetall ist, 1, und für den Fall, dass M ein Erdalkalimetall ist, 2 bedeutet, **dadurch gekennzeichnet, dass** n-Butyraldehyd, Formaldehyd und Base nach dem anorganischen Cannizzaro-Verfahren umgesetzt werden und man das entstehende Reaktionsgemisch, der Dampfdruckfiltration unterwirft.

2. Verfahren nach Anspruch 1, **dadurch gekennzeichnet, dass** M für Natrium oder Calcium steht.

3. Verfahren nach wenigstens einem der Ansprüche 1 bis 2, **dadurch gekennzeichnet, dass** das isolierte Formiatsalz einen Restfeuchtegehalt <3,5 Gew.-% aufweist.

4. Verfahren nach wenigstens einem der Ansprüche 1 bis 3, **dadurch gekennzeichnet, dass** die Dampfdruckfiltration kontinuierlich durchgeführt wird.

5. Verfahren nach wenigstens einem der Ansprüche 1 bis 4, **dadurch gekennzeichnet, dass** der Differenzdruck bei der Dampfdruckfiltration 0 bis 6 bar beträgt.

6. Verfahren nach wenigstens einem der Ansprüche 1 bis 5, **dadurch gekennzeichnet, dass** der sich bei der Dampfdruckfiltration bildende Filterkuchen während des Filtrationsvorgangs mindestens einem Waschvorgang unterworfen wird.

7. Verfahren nach wenigstens einem der Ansprüche 1 bis 6, **dadurch gekennzeichnet, dass** die Kuchenwäsche mit einer ganz oder teilweise gesättigten wäßrigen Lösung des zu isolierenden Formiatsalzes erfolgt.

8. Verfahren nach mindestens einem der Ansprüche 1 bis 7, **dadurch gekennzeichnet, dass** vom entstehenden Reaktionsgemisch vor der Abtrennung des Formiatsalzes destillierbare Bestandteile teilweise abgetrennt werden.

9. Verfahren nach mindestens einem der Ansprüche 1 bis 8, **dadurch gekennzeichnet, dass** bei der Dampfdruckfiltration die 4 Phasen Kuchenbildung, Kuchenwaschung, Kuchenbedampfung und Kuchenentfeuchtung durchlaufen werden.

10. Verfahren nach Anspruch 9, **dadurch gekennzeichnet, dass** bei der Kuchenbedamfung der auf der Filterfläche gebildete Filterkuchen aus dem abzutrennenden Formiatsalz mit einem dampfförmigen Behandlungsfluid beaufschlagt wird, dessen Druck und Temperatur derart eingestellt werden, dass das Behandlungsfluid im Filterkuchen kondensiert, vorzugsweise zunächst in der obersten, von der Filterfläche abgewandten Schicht des Filterkuchens, so dass entlang dieser Schicht eine Kondensationsfront gebildet wird und dass weiterhin der Filterkuchen von der obersten Schicht aus in Richtung der Filterflächen auf die Temperatur des gasförmigen Behandlungsfluids fortschreitend erwärmt wird, so dass dabei die Kondensationsfront an der Phasengrenze zwischen dem gasförmigen Behandlungsfluid und der Flüssigkeit zur Filterfläche hin verschoben wird.

11. Verfahren nach mindestens einem der Ansprüche 9 bis 10, **dadurch gekennzeichnet, dass** bei der Kuchenentfeuchtung der auf der Filterfläche gebildete Filterkuchen aus dem abzutrennenden Formiatsalz mit komprimiertem Inertgas und/oder komprimierter Luft behandelt wird.

12. Verfahren nach mindestens einem der Ansprüche 1 bis 11, **dadurch gekennzeichnet, dass** das Reaktionsgemisch nach der Dampfdruckfiltration mindestens einem weiteren Reinigungs- und Trocknungsschritt unterzogen wird, der eine Umkristallisation aus und/oder eine Redispergierung in einem Lösungsmittel beinhaltet.

13. Verfahren nach Anspruch 12, **dadurch gekennzeichnet, dass** es sich bei dem Lösungsmittel um Wasser oder ein Wasser enthaltendes Gemisch handelt.

## Claims

1. Process for preparing trimethylolpropane with simultaneous formation of formate salts of the formula
M(OOCH)ₙ (I),
where M represents the alkali metals lithium, sodium, potassium, rubidium, caesium and/or the alkaline earth metals beryllium, calcium, strontium, barium and n is 1 when M is an alkali metal and is 2 when M is an alkaline earth metal, **characterized in that** n-butyraldehyde, formaldehyde and a base are reacted by the inorganic Cannizzaro process and the reaction mixture formed is subjected to vapour pressure filtration.

2. Process according to Claim 1, **characterized in that** M represents sodium or calcium.

3. Process according to at least one of Claims 1 and 2, **characterized in that** the formate salt isolated has a residual moisture content of <3.5% by weight.

4. Process according to at least one of Claims 1 to 3, **characterized in that** the vapour pressure filtration is carried out continuously.

5. Process according to at least one of Claims 1 to 4, **characterized in that** the differential pressure in the vapour pressure filtration is from 0 to 6 bar.

6. Process according to at least one of Claims 1 to 5, **characterized in that** the filter cake formed in the vapour pressure filtration is subjected to at least one washing step during the filtration procedure.

7. Process according to at least one of Claims 1 to 6, **characterized in that** washing of the cake is carried out using a fully saturated or partially saturated aqueous solution of the formate salt to be isolated.

8. Process according to at least one of Claims 1 to 7, **characterized in that** distillable constituents are partly separated from the resulting reaction mixture prior to the formate salt being separated off.

9. Process according to at least one of Claims 1 to 8, **characterized in that** the 4 phases cake formation, cake washing, vapour treatment of the cake and demoisterizataion of the cake are carried out during the vapour pressure filtration.

10. Process according to Claim 9, **characterized in that**, in the vapour treatment of the cake, the filter cake of formate salt to be separated off formed on the filter area is treated with a gaseous treatment fluid whose pressure and temperature are set so that the treatment fluid condenses in the filter cake, preferably initially in the uppermost layer of the filter cake farthest from the filter area, so that a condensation front is formed along this layer and that, in addition, the filter cake is heated to the temperature of the gaseous treatment fluid progressively from the uppermost layer in the direction of the filter area, so that the condensation front at the phase boundary between the gaseous treatment fluid and the liquid is shifted towards the filter area.

11. Process according to at least one of Claims 9 and 10, **characterized in that**, in the demoisturization of the cake, the filter cake of formate salt to be separated off formed on the filter area is treated with compressed inert gas and/or compressed air.

12. Process according to at least one of Claims 1 to 11, **characterized in that** the reaction mixture after the vapour pressure filtration is subjected to at least one further purification and drying step which comprises a recrystallization from and/or redispersion in a solvent.

13. Process according to Claim 12, **characterized in that** the solvent is water or a water-containing mixture.

## Revendications

1. Procédé pour la préparation du triméthylolpropane avec formation simultanée de sels de l'acide formique répondant à la formule
M(OOCH)ₙ (I),
dans laquelle M représente les métaux alcalins, lithium, sodium, potassium, rubidium, césium et/ou les métaux alcalino-terreux, beryllium, calcium, strontium, baryum et n est égal à 1 lorsque M représente un métal alcalin et à 2 lorsque M représente un métal alcalino-terreux, **caractérisé en ce que** l'on fait réagir l'aldéhyde n-butyrique, le formaldéhyde et une base par le procédé inorganique de Cannizzaro et on soumet le mélange formé dans la réaction à une filtration sous pression de vapeur.

2. Procédé selon la revendication 1, **caractérisé en ce que** M représente le sodium ou le calcium.

3. Procédé selon au moins une des revendications 1 à 2, **caractérisé en ce que** le sel de l'acide formique isolé a une teneur en humidité résiduelle inférieure à 3,5 % en poids.

4. Procédé selon au moins une des revendications 1 à 3, **caractérisé en ce que** la filtration sous pression de vapeur est réalisée en continu.

5. Procédé selon au moins une des revendications 1 à 4, **caractérisé en ce que**, à la filtration sous pression de vapeur, la différence de pression est de 0 à 6 bars.

6. Procédé selon au moins une des revendications 1 à 5, **caractérisé en ce que** le gâteau de filtration qui se forme à la filtration sous pression de vapeur est soumis à au moins une opération de lavage au cours de l'opération de filtration.

7. Procédé selon au moins une des revendications 1 à 6, **caractérisé en ce que** le lavage du gâteau de filtration est réalisé avec une solution aqueuse partiellement ou totalement saturée du sel de l'acide formique à isoler.

8. Procédé selon au moins une des revendications 1 à 7, **caractérisé en ce que**, à partir du mélange formé dans la réaction et avant séparation du sel de l'acide formique, on sépare en partie les constituants distillables.

9. Procédé selon au moins une des revendications 1 à 8, **caractérisé en ce que**, à la filtration sous pression de vapeur, on passe par les quatre phases : formation du gâteau, lavage du gâteau, application de vapeur sur le gâteau et déshumidification du gâteau.

10. Procédé selon la revendication 9, **caractérisé en ce que**, à l'application de vapeur sur le gâteau, on traite le gâteau de filtration du sel de l'acide formique à séparer formé sur la surface filtrante par un fluide à l'état de vapeur dont la pression et la température sont réglées en sorte que ce fluide se condense dans le gâteau de filtration, de préférence d'abord dans la couche supérieure, la plus éloignée de la surface filtrante, du gâteau de filtration, en formant dans cette couche un front de condensation et que, ensuite, le gâteau de filtration, de la couche la plus éloignée de la surface filtrante vers cette surface, soit progressivement chauffé à la température du fluide de traitement gazeux, le front de condensation progressant à la limite de phase entre le fluide de traitement gazeux et le liquide vers la surface filtrante.

11. Procédé selon au moins une des revendications 9 à 10, **caractérisé en ce que**, à la déshumidification, le gâteau de filtration du sel de l'acide formique à séparer formé sur la surface filtrante est traité par un gaz inerte comprimé et/ou de l'air comprimé.

12. Procédé selon au moins une des revendications 1 à 11, **caractérisé en ce que** le mélange de réaction, après la filtration sous pression de vapeur, est soumis à au moins une autre opération de purification et de séchage, comprenant une recristallisation dans un solvant et/ou une redispersion dans un solvant.

13. Procédé selon la revendication 12, **caractérisé en ce que** le solvant consiste en eau ou en un mélange contenant de l'eau.
